Europäisches Patentamt

European Patent Office

Office européen des brevets

(19)

(11) Publication number: **0 194 552**
**A1**

(12) **EUROPEAN PATENT APPLICATION**

(21) Application number: **86102802.5**

(22) Date of filing: **04.03.86**

(51) Int. Cl.⁴: **C07C 1/04** , **B01J 23/78**

(30) Priority: **07.03.85 US 709157**

(43) Date of publication of application:
**17.09.86 Bulletin 86/38**

(84) Designated Contracting States:
**BE DE FR GB IT NL**

(71) Applicant: **AIR PRODUCTS AND CHEMICALS, INC.**
**P.O. Box 538**
**Allentown, Pennsylvania 18105(US)**

(72) Inventor: **Dyer, Paul Nigel**
**3920 Pleasant Avenue**
**Allentown, PA 18105(US)**
Inventor: **Pierantozzi, Ronald**
**1317 Butternut Lane**
**Macungie, PA 18062(US)**

(74) Representative: **Kador & Partner**
**Corneliusstrasse 15**
**D-8000 München 5(DE)**

(54) Selective conversion of synthesis gas.

(57) Process for selectively converting CO-rich synthesis gas to hydrocarbons in the diesel fuel carbon number range, preferably in an otherwise conventional slurry phase reaction, by utilization of a catalyst prepared by co-precipitation of cupric and ferric hydroxide, followed by alkaline salt impregnation and activation of the catalyst specie without intervening calcination. Omission of the calcination step, prior to activation, differentiates the catalyst of the present invention from the prior art, wherein all known teachings suggest that such calcination is necessary to enhance catalyst selectivity and activity.

EP 0 194 552 A1

## SELECTIVE CONVERSION OF SYNTHESIS GAS

This invention was made under DOE contract number DE-AC22-80PC30021 and is subject to government rights arising therefrom.

### TECHNICAL FIELD

This invention relates to a Fischer-Tropsch catalyzed process for the selective conversion of synthesis gas to hydrocarbons with a disproportionately high concentration of products in the diesel fuel range and to catalysts therefor.

### BACKGROUND OF THE INVENTION

Production of hydrocarbon liquid fuels from coal may be accomplished, in part, by the Fischer-Tropsch catalyzed synthesis of hydrocarbons from synthesis gas produced by coal gasification. The Fischer-Tropsch synthesis (or conversion), however, is unselective in nature, producing molecules with carbon numbers typically in the range 1-40 in proportions controlled by process kinetics. Process efficiency would be enhanced by selective conversion of the synthesis gas to relatively narrow carbon number range hydrocarbons, for example, a general range of $C_9$-$C_{25}$ hydrocarbons for the selective production of diesel fuel.

Fischer-Tropsch conversion of synthesis gas has heretofore been conducted both in fixed bed, gas-solid reactors, gas entrained fluidized bed reactors, and in slurry phase reactors. The former is described by H. H. Storch et al in "The Fischer-Tropsch and Related Syntheses," Wiley, 1951, while the latter is described by Kolbel et al (Catalysis Review Science and Engineering, 1980, 21, 225), Poutsma (ORNL-5635, 1980, "Assessment of Advanced Process Concepts for the Liquefaction of Low $H_2$/CO Ratio Synthesis Gas") and Deckwer et al (Industrial Engineering Chemical Process Design Developments, 1982, Volume 21, pages 222 and 231). The latter references in particular indicate the potential incentive for using high CO/$H_2$ ratio synthesis gas (sometimes referred to as "syngas") in liquid phase slurry reactors. More recently, Satterfield et al (Industrial Engineering Chemical Process Design Developments, 1982, Volume 21, page 456) described literature on product distribution in Fischer-Tropsch synthesis, particularly slurry reactors using iron catalysts. Collectively, analyses indicate that the product selectivity in such syntheses follows a distribution, predicted by the Schulz-Flory model, characterized by a chain growth probability factor (alpha), and that any reported deviations in publications concerning the Fischer-Tropsch process are probably due to experimental artifacts.

The maximum fuel product fractions predictable from the Schulz-Flory distribution, together with an indication of methane and $C_{26+}$ fraction are as follows:

Schulz-Flory Maxima

Gasoline Range

| Alpha | $C_1$ wt. % | $C_{5-11}$ wt. % | $C_{9-25}$ wt. % | $C_{26+}$ wt. % |
|---|---|---|---|---|
| 0.76 | 5.8 | 47.6 | 31.8 | 0.7 |

Diesel Range

| Alpha | | | | |
|---|---|---|---|---|
| 0.88 | 1.4 | 31.9 | 54.1 | 12.9 |

In general, prior publications indicate that most Fischer-Tropsch (FT) products adhere to the Schulz-Flory (SF) distribution, regardless of catalyst type.

This unselective type of polymerization process is represented by a straight line on a plot of log $(W_i/C_i)$ against $C_i$, and has significant implications for the maximum yield of transportation fuel product fractions, such as diesel fuel wherein polymerization to carbon number hydrocarbons in the range of $C_5$-$C_{25}$ and particularly $C_9$-$C_{25}$ are preferred.

Satterfield et al specifically concluded that the distribution generally held for iron based FT catalyst with values of alpha ranging from 0.55 to 0.94. Short term success in selective syngas conversion, by utilization of a proposed metal particle size effect or by use of $Co_2(CO_8)$ on alumina of selective pore size distribution (Blanchard, J. C. S. Chem Comm., 1979, page 605, or catalysts comprised of Ruthenium in Y zeolites, for example, has been reported by Nijs et al (Journal of Catalysis, 1980, Volume 65, page 328. However, none of these previous attempts has produced a stable selectivity over a period of time, the observed selectivity in each case disappearing over a period of hours and reverting to the expected Schulz-Flory distribution.

One prior publication particularly pertinent to the present invention is The Fischer-Tropsch Syntheses In the Liquid Phase, Kolbel and Ralek, Catalysis Review of Science Engineering, 1980, 21 (2), 225-274. Kolbel, et al, in particular, describe a Fischer-Tropsch catalyzed reaction to produce hydrocarbon fuels from synthesis gas, utilizing a catalyst comprising co-precipitated iron and copper impregnated with potassium. Kolbel et al have also described such a catalyst made from a catalyst precursor prepared using known technology by precipitation at controlled pH and temperature in a stirred, tubular flow-through reactor, much like the catalyst preparation procedure in the present invention.

Kolbel et al also suggested however, "activity, selectivity, efficiency, lifetime...are increased considerably if, after drying, the ferric oxide catalyst is subjected to annealing at 250° -350°C with air or oxygen" (Kolbel at 245). The Kolbel et al reference in fact neither suggests nor discloses a process which uses a "non-annealed" or "non-calcined" (the terms are believed to be interchangeable) form of that catalyst.

So far as is known, all others, as well as Kolbel et al, who have utilized such a catalyst have done so with the catalyst in calcined form, in the manner described by Kolbel et al.

The objective of the present invention is to improve the selectivity of a Fischer-Tropsch catalyzed synthesis gas conversion reaction to hydrocarbon fuel product within relatively narrow carbon number ranges, particularly in the $C_5$ - $C_{25}$ diesel fuel range.

A more particular object of the present invention is to provide such a reaction, the selectivity and stability of which is improved by the utilization of an improved catalyst.

## SUMMARY OF THE INVENTION

In accordance with the present invention, synthesis gas, and particularly CO-rich syngas (i.e. $CO:H_2$ ratio of 0.5:1 to 2:1 and particularly 1:1 to 1.5:1) is subjected to Fischer-Tropsch catalysis, preferably in a conventional slurry phase reactor with a stable enhanced selectivity catalyst formulated as follows:

A catalyst precursor is prepared using known technology by precipitation at controlled pH and temperature in a stirred tubular flow-through reactor. Using this technique, solutions of cupric and ferric ions, preferably a ferric nitrate and a cupric nitrate solution are continuously precipitated by reaction with alkaline solution, particularly an alkaline hydroxide such as potassium, lithium, sodium or ammonium hydroxide. The co-precipitate is formed from solution at a pH of 5-9 preferably about 7, and a temperature from about 50°C up to the boiling point of the solution, preferably about 80-95°C. The co-precipitate is filtered and washed and then impregnated with a solution of a Group 1A metal - (sodium, potassium, cesium, rubidium or lithium) salt, specifically a salt of an anion which is not poisonous to the catalyst. Such anions include acetate, nitrate, carbonate and bicarbonate. The preferred salt is potassium bicarbonate. The general chemical notation for this product is Fe/Cu/M, in which M is a Group 1A metal.

The impregnated co-precipitated product is dried below 250°C, preferably 105-125°C, in air or an inert gas mixture. Calcining of this product is avoided both in the drying step and in the activation step which follows. The dried product, produced as described above, is then activated for use as a catalyst in the Fischer-Tropsch reaction.

In order to activate the catalyst, it is first heated in an inert gas such as nitrogen and/or helium to a temperature on the order of 100-240°C (preferably 170-190°C), for a period of time of 1-24 hours. With the catalyst at this temperature, it is then activated by exposure to a reducing atmosphere at still higher temperatures and elevated pressure. More specifically, the catalyst is heated over a period of time on the order of .14-60 hours, preferably about 8-20 hours, in stages to the operating temperature and pressure of the catalytic reaction, typically on the order of 300 psig and 240°C. The reducing gas to which the catalyst is exposed for this purpose is generally a hydrogen-containing gas and may be the same synthesis gas used in a subsequent reaction, typically with a $CO/H_2$ mole ratio on the order of 0.5:1 to 2:1, preferably on the order of about 0.9:1 to 1.6:1.

In the same or a similar reducing atmosphere, the catalyst is then reacted with the $CO-H_2$ mixture to form the hydrocarbon products. Preferably activation occurs in the same reactor as and immediately preceeding the catalyzed reaction process itself.

The catalyzed reaction is preferably a slurry phase reaction with a synthesis gas preferably in the following parameter ranges:

| | $CO:H_2$ Ratio | Pressure psig | Temp.°C | GHSV* Hours$^{-1}$ |
|---|---|---|---|---|
| Broad Range | 0.5:1-2:1 | 100-600 | 200-280 | 100-2000 |
| Preferred Range | 0.9:1-1.6:1 | 150-500 | 220-270 | 150-2000 |
| Most Preferred Range | 0.9:1-1.6:1 | 150-500 | 235-260 | 150-1000 |

\* Gas Hourly Space Velocity
(Volume gas/Volume slurry/Hour)

For a more complete understanding of the present invention, reference may be made to the following detailed description thereof, taken together with the subjoined claims.

BRIEF DESCRIPTION OF THE FIGURES

Figure 1 depicts product distributions obtained utilizing the method of the present invention (Example 8) as compared to that of the prior art (Comparative Example 9).

Figure 2 illustrates the product distribution obtained in a slurry phase reactor (Example 7), with the same catalyst batch prepared by the method of the present invention, as used in the fixed bed reactor experimental run illustrated in Figure 1 (Example 8).

Figure 3 illustrates product distributions in two experimental runs (Examples 6 and 5) in a slurry reactor at higher $CO/H_2$ in accordance with the present invention; and

Figure 4 is a graphical illustration of the Schulz-Flory distribution for two experimental runs utilized in the process of the present invention (Examples 5 and 6), and a comparison run (Comparative Example 2) utilizing a typical prior art catalyst ($Fe_2O_3$).

DETAILED DESCRIPTION OF THE INVENTION

Normally, a catalyst of the type used in the present invention, is calcined in air at 250-500°C, before reduction to an active species for use in the Fischer-Tropsch catalyzed conversion of synthesis gas to hydrocarbon fuels. It has been discovered that product selectivity is greatly improved if the catalyst is not calcined in air after the drying stage. Instead, the catalyst is activated without prior calcination. Most preferably, the catalyst is transferred directly to the reactor, as a solid in the case of a fixed bed reactor, or as a suspension in oil for the slurry reactor.

The catalyst is then activated in situ by heating, for example, to 180°C in an inert atmosphere, such as nitrogen, and then contacting the catalyst with a hydrogen-containing reducing gas, such as a $CO/H_2$ mixture (in a mole ratio range of 0.5:1 to 2:1, preferably about 1:1) at one atmosphere pressure, followed by a slow increase in temperature and pressure to the operating conditions of the reaction, typically 240°C and 300 psig.

For the preferred reaction in a mixed slurry phase, the range of reaction conditions are generally 150 -500 psig, 220 -270°C and a space velocity of 150-2000 hours⁻¹.

Similarly, the process of this invention may be conducted with a fixed (solid) or fluidized (including gas entrained) bed of catalyst. For this alternative, parameter ranges are:

| | $CO/H_2$ Ratio | Pressure (psig) | Temp. (°C) | GHSV* (Hours-1) |
|---|---|---|---|---|
| Range | 0.5:1-2:1 | 100-600 | 200-350 | 100-5000 |
| Preferred | | 150-500 | 200-280 | 150-1000 |
| Most Preferred | 0.9:1-1.6:1 | 150-500 | 220-260 | 180-650 |

* Volume gas/Volume catalyst bed/hour

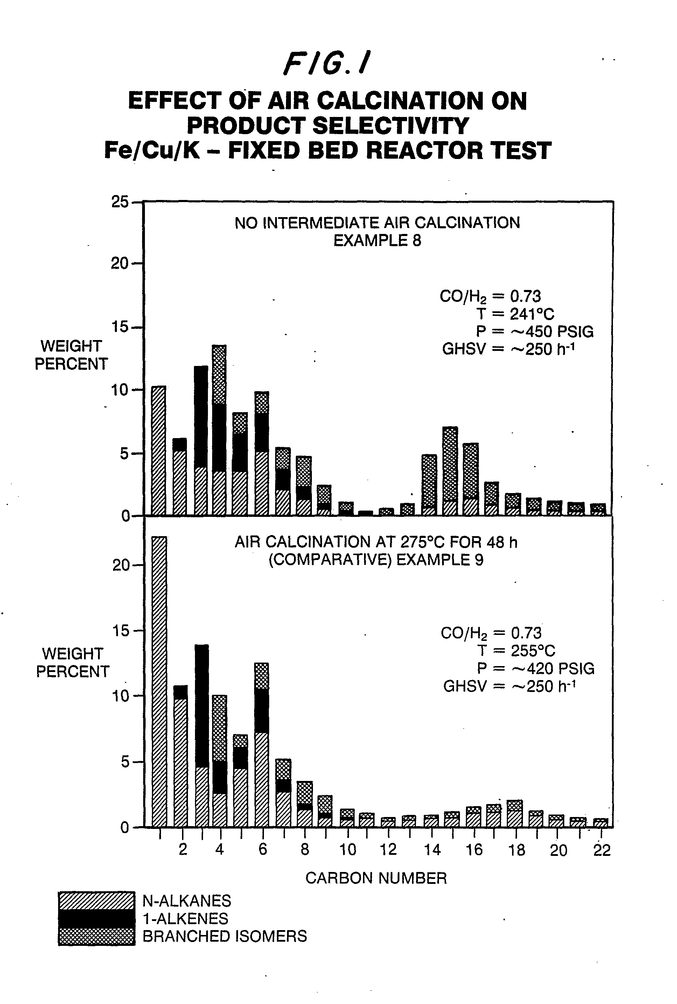

Figure 1 indicates the enhancement of product selectivity that results from this type of treatment and activation - (Example 8) compared with an intermediate calcination in air, of the same catalyst, at 275°C for 48 hours (Example 9) between the drying and activation stages. Specifically, Figure 1 illustrates results from a fixed bed reactor, under conditions which produced a high pressure drop and plugging within a few hours, when the catalyst of Example 8 was used. This plugging is due to the enhanced selectivity to higher molecular weight products.

In the slurry phase, however, it has been possible to operate the catalyst of the present invention continuously for up to about 1900 hours with minimal catalyst deactivation and no loss of product selectivity at pressures between 150 -500 psig and temperatures of 235 -260°C and space velocities of 150-1000 hours ⁻¹ with $CO/H_2$ ratio syngas in the range 0.9:1 to 1.6:1. In these tests, any higher molecular weight material that did not distill over with the product stream, but built up in the reactor, was removed via a filtered sidestream, and then included in the overall product distribution.

EXAMPLES 1-9

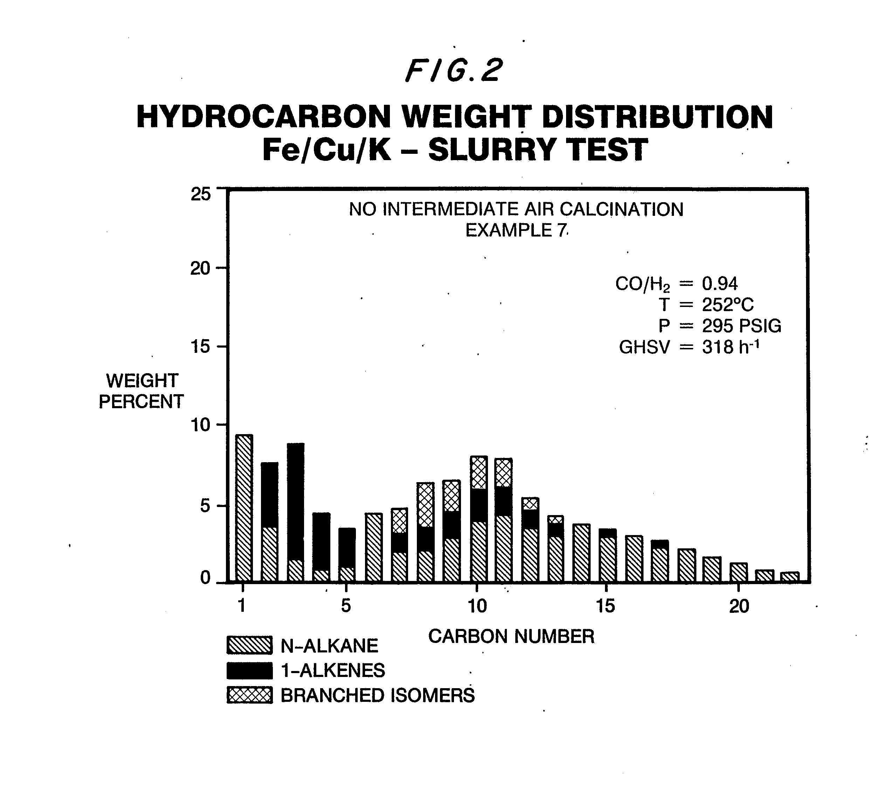

Typical results obtained with the catalyst prepared according this invention, and certain prior art comparative catalysts, are summarized in Table 1, set forth as Examples 1-9 of which 1-3 and 9 are Comparative Examples. Some of the data for Examples 2 and 5-7 are illustrated in Figures 2 to 4. Figure 2 illustrates the product distribution of Example 7 (a slurry phase reaction). Product selectivity was reproduced, although centered at $C_{10}$ compared with $C_{15}$ in the fixed bed test (Example 8), possibly due to the difference between the continuous nature of the slurry test and the limiting conditions of the fixed bed run. Examples 7 and 8 utilized catalyst from a common batch.

Table 2 illustrates elemental analyses of the catalysts used in Examples 4-9. Analytical data is presented for the catalyst in both its dried (but unreduced) form--referred to as "unreacted"--and after reaction. In general these catalysts were formulated in a procedure designed to give a metal ion ratio on the order of 100:0.5:0.2 iron: copper: potassium carbonate. In its dried and unreduced form, the catalyst is thought to consist primarily of ferric oxide hydrates and hydroxides with small amounts of copper hydroxide and potassium carbonate. Following similar formulation procedures, it is thought that workable catalyst can be produced within a metallic ion concentration range as follows:

Iron, at least 50% by wt., preferably at least 65% by wt.

Copper -0.01-5 wt.%, preferably 0.1-2% and most preferably 0.3-0.7%).

Group IA metal -generally 0.01-5.0%, preferably 0.1-1% and most preferably 0.05-0.2%.

It is important that the catalyst be formed at essentially constant pH and temperature conditions. For that purpose, a stirred flow-through reactor is preferred. Using such reactor, with resistive wall heaters thermostatically controlled and reactants and reactor preheated to an essentially constant temperature on the order of 90°C, the catalyst referred to in the examples herein were made in general by feeding to the reactor dilute solutions of ferric nitrate and cupric nitrate at rates selected to produced a molar mixture in the reactor of cupric and ferric ions in the proportions desired in the final product, namely 100:0.5. At the same time, ammonium hydroxide was fed at a rate controlled by a pH sensor to maintain the pH essentially constant at about 7. Precipitate removed from outflowing solution mixture was immediately vacuum filtered and washed. Thereafter it was dried and activated in accordance with the process description set forth above.

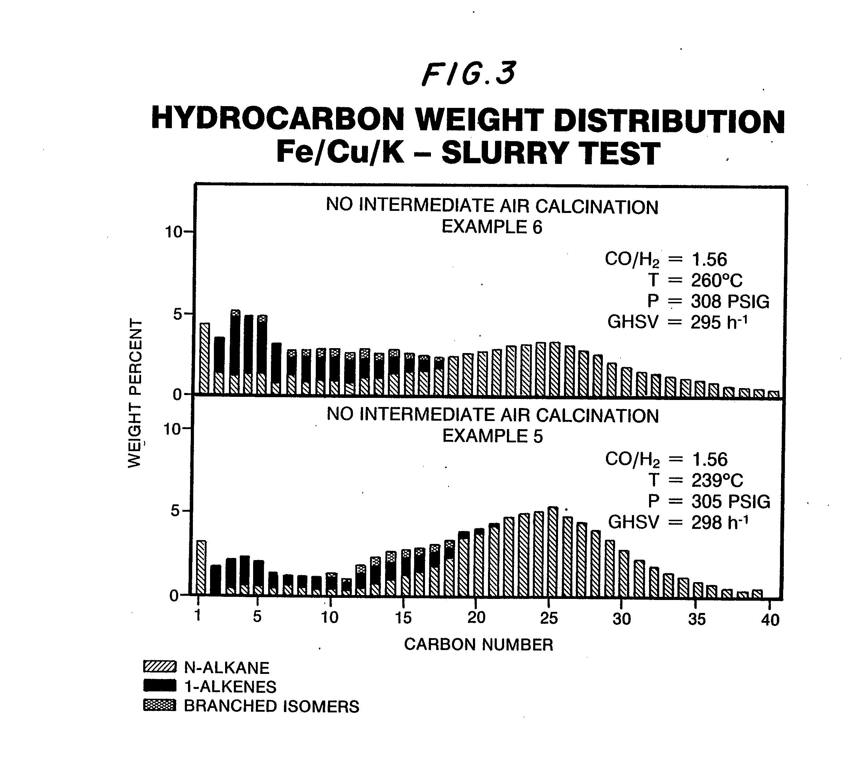

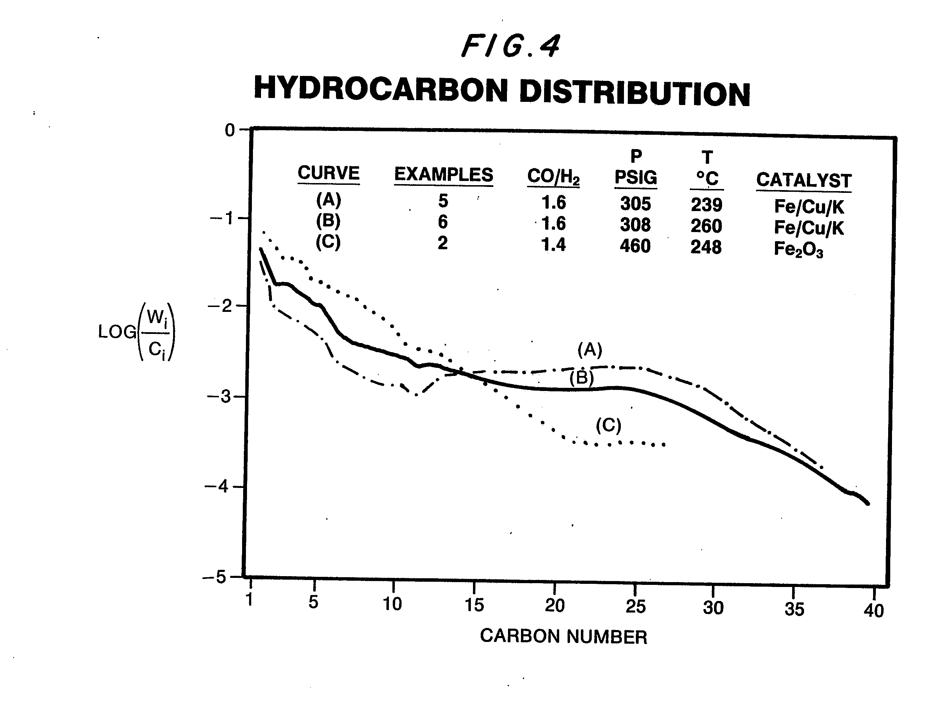

Typical results for Examples 5 and 6 are illustrated in Figures 3 and 4. They show low $CH_4$ yields and good selectivity for higher molecular weight products at high "time on stream." For example, the data illustrative of Example 5 in Figure 3 is taken at 660 hours (26 days) after the beginning of the run.

In Figure 4, deviations from a standard Schulz-Flory distribution are shown by reference to data from Examples 5 and 6, as compared to results from a standard sintered $Fe_2O_3$ baseline catalyst (Example 2) which conforms closely to a Schulz-Flory distribution for comparison. These results show that while the selectivity range is subject to some variation, centering between $C_{10}$ to $C_{25}$, catalysts prepared in accordance with the present invention consistently show enhanced selectivity in the $C_9$-$C_{25}$ range.

In the slurry tests, deactivation rates were low, and the catalyst was more active than the standard, sintered iron oxide catalyst (Comparative Examples 1 and 2).

For comparison, following is a brief discussion of the prior work reported by Kolbel et al. Co-precipitated Fe/Cu/K was used as a catalyst by Kolbel and Ralek in the 1.6 meter id x 8.6 meter high pilot plant, three phase bubble column reactor at Rheinpreussen in Germany, and possibly also in bench scale column experiments. The activation method Kolbel employed was to heat the slurry rapidly under $N_2$ (or $CO_2$) at operating pressure to 15 to 30°C above operating temperature, and then switch suddenly to 1.3 -1.5 $CO/H_2$ at high space velocity. After reduction was complete, as monitored by the exit $CO_2$, the temperature was lowered to the operating conditions.

Published data for Kolbel's slurry operations are listed in Table 3, which also compares typical product distributions obtained from commercial FT units at SASOL in South Africa. The operation at Rheinpreussen was geared to maximize the yield of gasoline. Some doubt exists about the overall product analysis, however, since the data reported in Table 3 was stated to be from a "sample from large storage tanks which contained the products from one of the longer operating periods." It may not, therefore, fully represent the percentage of light products obtained from the plant, which were removed by oil scrubbing and activated carbon absorption. With this in mind, a comparison of the product fractions in Table 3 indicates that previous slurry phase operations, together with the SASOL reactors, gave products which were generally unselective and tended to conform to the expected S-F distribution. The diesel fuel product fractions obtained by Kolbel are less than those expected from the S-F normal distribution.

Most importantly, calculations from the data in the Kolbel et al publication indicate that the calcined catalyst used by Kolbel et al deactivated and the reaction selectivity deteriorated relatively quickly. In contrast, the catalyst in the catalyzed process of the present invention is stable and maintains its selective catalytic activity over an extended period of time.

<div align="center">

TABLE I

COMPARATIVE CATALYST TEST DATA

</div>

| Catalyst | Phase | Example No. | Metal Wt% | Slurry Loading Wt% | P psig | T °C | GHSV h-1 | CO/H2 | Activity mol syngas/ kg cat/h | C1 | C5-C11 | C9-C25 | C26+ |
|---|---|---|---|---|---|---|---|---|---|---|---|---|---|
| Fe2O3 | slurry | 1 (Comp.) | 67.1* | 9.3 | 480 | 280 | 250 | 0.45 | 26.2 | 17.4 | 37.6 | 17.3 | 0.1 |
| | slurry | 2 " | 67.1* | 14.7 | 460 | 248 | 146 | 1.40 | 10.2 | 7.4 | 39.9 | 30.3 | 1. |
| | | | | | 455 | 277 | 145 | 1.39 | 20.8 | 6.8 | 41.1 | 36.2 | 2.7 |
| | | | | | 450 | 281 | 183 | 2.83 | 14.3 | 4.7 | 23.8 | 39.9 | 12.7 |
| | fixed bed | 3 " | 67.1* | | 455 | 253 | 295 | 0.73 | 6.3 | 15.9 | 46.2 | 17.8 | 0.1 |
| Fe/Cu/K (uncalcined) | slurry | 4 | 67.6* | 19.2* | 320 | 237 | 307 | 1.03 | 13.9 | 6.2 | 9.8 | 46.1[1] | 26.8 |
| | | | | | 165 | 241 | 298 | 0.96 | 11.3 | 4.3 | 23.7 | 44.5 | 23.4 |
| " | slurry | 5 | 66.2* | 16.9* | 300 | 237 | 299 | 1.01 | 15.7 | 2.9 | 11.5 | 49.8[2] | 30.5 |
| | | | | 16.9* | 305 | 239 | 298 | 1.56 | 15.7 | 3.1 | 9.5 | 55.4 | 29.4 |
| | | | | | 307 | 238 | 299 | 1.54 | 14.8 | 2.6 | 7.5 | 50.5 | 30.3 |
| | | | | 18.0* | 303 | 255 | 332 | 1.54 | 26.5 | 6.0 | 25.7 | 48.6 | 10 |
| | | | | | 310 | 260 | 371 | 1.54 | 32.5 | 6.9 | 30.9 | 47.4 | 7.8 |
| | | | | 19.8* | 305 | 257 | 185 | 1.82 | 21.8 | 6.9 | 40.0 | 37.5 | 10.5 |
| " | slurry | 6 | 64.1* | 19.8* | 308 | 260 | 295 | 1.56 | 32.2 | 4.3 | 22.4 | 48.1 | 20.0 |
| " | slurry | 7 | 67.2* | 15.5* | 295 | 252 | 318 | 0.94 | 9.9 | 9.5 | 41.3 | 50.8 | 0.1 |
| (3) | fixed bed | 8 | 67.2* | — | 450 | 241 | 250 | 0.73 | 9.3 | 10.2 | 31.5 | 30.4 | 0.1 |
| | fixed bed | 9 (Comp.) | 68.3* | — | 420 | 255 | 250 | 0.73 | 15.6 | 22.2 | 32.3 | 15.4 | 0.1 |

* unreduced catalyst basis    (1)  64.0% $C_{18}-C_{35}$   (2)  57.7% $C_{18}-C_{35}$   (3)  Fe/Cu/K Calcined 275°C, 48 hours

0 194 552

## TABLE II

### ELEMENTAL ANALYSES OF COPRECIPITATED Fe/Cu/K

| Example No. | Unreacted wt. % | | | After Reaction wt. % | | | | |
|---|---|---|---|---|---|---|---|---|
| | Fe | Cu | K | Fe | Cu | K | C | H |
| 8 | 67.2 ±1.3 | 0.4 ±0.1 | 0.08 ±0.01 | 62.4 ±1.3 | 0.7 ±0.1 | 0.13 ±0.02 | 9.28 | 0.53 |
| 9 | 68.3 ±1.4 | 0.4 ±0.1 | 0.05 ±0.01 | 64.2 ±1.3 | 0.4 ±0.1 | 0.05 ±0.01 | 5.49 | 0.54 |
| 7 | 67.2 ±1.3 | 0.4 ±0.01 | 0.08 ±0.01 | *81.3 ±1.1 | 0.47 ±0.01 | 0.11 ±0.01 | 8.90 | 0.15 |
| 4 (phase 1) | 69.11 | 0.43 | 0.09 | *67.0 ±1.0 | 0.40 ±0.01 | 0.05 ±0.01 | 1.71 | 0.17 |
| 5 (phase 2) | 66.20 | 0.47 | 0.18 | 67.3 ±1.0 | 0.49 ±0.02 | 0.12 ±0.01 | | |
| 6 (phase 3) | 64.10 | 0.38 | 0.12 | | | | | |

* Slurry extracted under $N_2$ with (a) toluene for 72 h, (b) hexane for 24 h

TABLE III

### A COMPARISON OF COMMERCIAL SASOL AND PRIOR SLURRY PHASE FISCHER-TROPSCH DATA

| Reaction | Sasol | | Rheinpreussen | Kolbel Bench Scale Slurry | | |
| --- | --- | --- | --- | --- | --- | --- |
| | Arge fixed bed | Synthol gas entrained solid | slurry 1.6 id x 8.6 m | "Low MW" *** | "Medium MW" *** | "High MW" *** |
| $CO/H_2$ | 0.6 | 0.5 | 1.5 | 1.5 | | |
| T °C | 220-250 | 320 | 268 | 268 | | |
| P MPa | 2.6 | 2.0 | 1.2 | 1.1 | | |
| Conversion % | 70 | 85 | 89 | 88 | | |
| Recycle | 2.5 | 2 | 0 | 0 | | |
| Space Time Yield $kg/m_3/h$ | 50-70 | 90 | 41 | 32 | | |
| $C_1$ Wt.% | 5 | 10 | + 6.8 $(C_1 + C_2)$ | | | |
| $C_3-C_4$ | | | | 18.0 | 6.9 | 2.2 |
| Approx. Gasoline Range Wt.% | 22 | 39 $(C_5-C_{12})$ | 53.6 (40-180°C) | 68.1 | 40.0 | 7.1 $(C_5 - 190°C)$ |
| Approx. Diesel Range Wt.% | 21 | 6 $(C_{13}-C_{21})$ | 10-12 (180-320°C) | 13.3 | 44.0 | 41.2 (190-450°C) |
| Higher Hydrocarbon Wt.% | 35 | 5 $(C_{22}+)$ | 1.9 (½320°C) | 0.6 | 9.1 | 49.5 (½450°C) |

+ Storage tank sample

\* Wt.% of $C_3+$ products. Unreported amounts of $C_1-C_2$ make determination of approximate diesel range inaccurate.

*** As designated by Kolbel

## STATEMENT OF INDUSTRIAL UTILITY

Utilization of a co-precipitated Fe/Cu/M (M = Group 1A metal) catalyst as taught herein enhances the overall thermal efficiency of the indirect liquefaction process for producing hydrocarbon fuels from coal. The yield of desired liquid fuel fraction in a single pass, particularly in the diesel fuel range, is enhanced and this improves the integration of the second stage Fischer-Tropsch reaction with the initial gasification step. Moreover, the selective catalyst used in the process of the present invention is stable over an extended period of time, further contributing to the efficiency of the present invention.

## Claims

1. In the method of converting synthesis gas, including CO and $H_2$ in a mole ratio of 0.5:1 to 2:1, to $C_9$ -$C_{25}$ hydrocarbons by Fischer-Tropsch reaction, the improvement consisting of conducting said reaction in the presence of a non-calcined catalyst produced by co-precipitation of ferric and cupric hydroxide, and then impregnating said co-precipitate with a Group IA metal salt.

2. An improved method, as recited in Claim 1, wherein said co-precipitation is conducted by reacting solutions of cupric and ferric nitrate with alkaline hydroxide and the coprecipitate produced thereby is filtered and washed prior to said impregnation with said salt.

3. An improved method, as recited in Claim 2, wherein said co-precipitation is conducted at about 90°C and pH 5-9.

4. An improved method, as recited in Claim 3, wherein said impregnated co-precipitate is dried at about 120°C in air or an inert atmosphere.

5. An improved method, as recited in Claim 2, wherein said dried, impregnated, co-precipitate is activated for said catalyzed reaction by exposure to an inert gas at 100-240°C for a period of 1-24 hours.

6. An improved method, as recited in Claim 5, wherein said activation process comprises an additional exposure to a hydrogen containing reducing gas.

7. An improved method, as recited in claim 6, wherein during said catalyst activation the pressure and temperature of the reducing gas are slowly increased to the desired temperature and pressure of said catalyzed reaction.

8. An improved method, as recited in Claim 7, wherein said reducing gas is a 0.5:1 to 2:1 mole ratio mixture of carbon monoxide and hydrogen.

9. An improved method, as recited in Claim 8, wherein said activation process comprises exposure to said inert gas followed by exposure to said reducing gas up to a temperature of 240°C and a pressure of 300 psig for about 8-20 hours.

10. An improved method, as recited in Claim 9, wherein said reducing gas is a 1:1 mole mixture of carbon monoxide and hydrogen and said activation is carried out in the same reactor and immediately preceding said catalyzed reaction.

11. An improved method, as recited in Claim 1, wherein the conditions for said reaction include:

| | |
|---|---|
| Catalyst phase | Solid or entrained gas |
| $CO:H_2$ mole ratio | 0.5:1 to 2:1 |
| Temperature | 200-350°C |
| Pressure | 100-600 psig |
| Space Velocity | 100-5000 hours$^{-1}$ |

12. An improved method, as recited in Claim 1, wherein the conditions for said reaction include:

| | |
|---|---|
| Catalyst phase | Solid or entrained gas |
| $CO:H_2$ mole ratio | 0.5:1 to 2:1 |
| Temperature | 200-280°C |
| Pressure | 150-500 psig |
| Space Velocity | 150-1000 hours$^{-1}$ |

13. An improved method, as recited in Claim 1, wherein the conditions for said reaction include:

| Catalyst phase | Solid or entrained gas |
| --- | --- |
| $CO:H_2$ mole ratio | 0.9:1 to 1.6:1 |
| Temperature | 220-260°C |
| Pressure | 150-500 psig |
| Space Velocity | 180-650 hours$^{-1}$ |

14. An improved method, as recited in Claim 1, wherein the conditions for said reaction include:

| Catalyst phase | Slurry |
| --- | --- |
| $CO:H_2$ mole ratio | 0.5:1 to 2:1 |
| Temperature | 200-280°C |
| Pressure | 100-600 psig |
| Space Velocity | 100-2000 hours$^{-1}$ |

15. An improved method, as recited in Claim 1, wherein the conditions for said reaction include:

| Catalyst phase | Slurry |
| --- | --- |
| $CO:H_2$ mole ratio | 0.9:1 to 1.6:1 |
| Temperature | 220-270°C |
| Pressure | 150-500 psig |
| Space Velocity | 150-2000 hours$^{-1}$ |

16. An improved method, as recited in Claim 1, wherein the conditions for said reaction include:

| Catalyst phase | Slurry |
| --- | --- |
| $CO:H_2$ mole ratio | 0.9:1 to 1.6:1 |
| Temperature | 235-260°C |
| Pressure | 150-500 psig |
| Space Velocity | 150-1000 hours$^{-1}$ |

17. An improved method, as recited in Claim 2, wherein Group 1A metal salt is potassium bicarbonate.

18. A method of making a catalyst adapted for the selective conversion of synthesis gas to $C_9$-$C_{25}$ hydrocarbons comprising co-precipitating ferric and cupric hydroxide, impregnating said coprecipitate with an alkaline salt and then, while avoiding calcination of said impregnated coprecipitate, drying and activating said coprecipitate by exposure to an inert gas followed by a reducing gas at elevated temperature and pressure.

19. A catalyst made in accordance with the method of claim 18.

20. A catalyst, as recited in claim 19, wherein the weight % elemental content of said catalyst, in dried unreduced form includes at least 50% iron, .01-5.0% of a Group IA metal and 0.01-5.0% copper.

21. A catalyst, as recited in claim 20, wherein the weight % elemental content of said catalyst, in dried unreduced form includes 0.1-1.0% of a Group IA metal and 0.1-2.0% copper.

22. A catalyst, as recited in claim 21, wherein the weight % elemental content of said catalyst, in dried unreduced form includes 0.2-0.05% of a GroupIA metal and 0.3-0.7% copper.

**0 194 552**

# FIG. 1

## EFFECT OF AIR CALCINATION ON PRODUCT SELECTIVITY
## Fe/Cu/K – FIXED BED REACTOR TEST

NO INTERMEDIATE AIR CALCINATION
EXAMPLE 8

$CO/H_2 = 0.73$
$T = 241°C$
$P = \sim450$ PSIG
$GHSV = \sim250$ h$^{-1}$

AIR CALCINATION AT 275°C FOR 48 h
(COMPARATIVE) EXAMPLE 9

$CO/H_2 = 0.73$
$T = 255°C$
$P = \sim420$ PSIG
$GHSV = \sim250$ h$^{-1}$

WEIGHT PERCENT

CARBON NUMBER

N-ALKANES
1-ALKENES
BRANCHED ISOMERS

# FIG.2

## HYDROCARBON WEIGHT DISTRIBUTION
## Fe/Cu/K – SLURRY TEST

NO INTERMEDIATE AIR CALCINATION
EXAMPLE 7

$CO/H_2 = 0.94$
$T = 252°C$
$P = 295$ PSIG
$GHSV = 318$ $h^{-1}$

WEIGHT PERCENT

CARBON NUMBER

N-ALKANE
1-ALKENES
BRANCHED ISOMERS

0 194 552

# FIG.3

## HYDROCARBON WEIGHT DISTRIBUTION
## Fe/Cu/K – SLURRY TEST

NO INTERMEDIATE AIR CALCINATION
EXAMPLE 6

$CO/H_2 = 1.56$
$T = 260°C$
$P = 308$ PSIG
$GHSV = 295$ $h^{-1}$

NO INTERMEDIATE AIR CALCINATION
EXAMPLE 5

$CO/H_2 = 1.56$
$T = 239°C$
$P = 305$ PSIG
$GHSV = 298$ $h^{-1}$

WEIGHT PERCENT

CARBON NUMBER

N-ALKANE
1-ALKENES
BRANCHED ISOMERS

0 194 552

## FIG.4

## HYDROCARBON DISTRIBUTION

| CURVE | EXAMPLES | $CO/H_2$ | P PSIG | T °C | CATALYST |
|-------|----------|----------|--------|------|----------|
| (A) | 5 | 1.6 | 305 | 239 | Fe/Cu/K |
| (B) | 6 | 1.6 | 308 | 260 | Fe/Cu/K |
| (C) | 2 | 1.4 | 460 | 248 | $Fe_2O_3$ |

y-axis: $LOG\left(\dfrac{W_i}{C_i}\right)$

x-axis: CARBON NUMBER

## DOCUMENTS CONSIDERED TO BE RELEVANT

EP 86102802.5

| Category | Citation of document with indication, where appropriate, of relevant passages | Relevant to claim | CLASSIFICATION OF THE APPLICATION (Int. Cl.4) |
|---|---|---|---|
| X | DE - C - 971 908 (RUHRCHEMIE)<br>* Example 1; claims 1-3 *<br>-- | 1-4,<br>11,18,<br>19 | C 07 C 1/04<br>B 01 J 23/78 |
| X | FR - A - 1 065 567 (RUHRCHEMIE)<br>* Examples 1,2 *<br>-- | 1,2,<br>11,18,<br>19 | |
| X | DE - C - 937 706 (RUHRCHEMIE)<br>* Example 1 *<br>-- | 1-4,<br>11,18,<br>19 | |
| X | DE - C - 919 289 (RUHRCHEMIE)<br>* Example 1 *<br>-- | 1-4,<br>11,18,<br>19 | |
| X | DE - C - 904 891 (RUHRCHEMIE)<br>* Examples 1,2 *<br>-- | 1,2,<br>11,18,<br>19 | **TECHNICAL FIELDS SEARCHED (Int. Cl.4)** |
| D,X | CATALYSIS REVIEWS-SCIENCE AND ENGENEERING, vol. 21, no. 2, 1980<br>H.KÖLBEL AND M.RALEK "The Fischer-Tropsch Synthesis in the liquid phase"<br>pages 225-274<br> * Page 244, line 6 - page 246, line 29; tables 2,11 *<br>-- | 1,2,5-<br>8,11,<br>12,14,<br>15,18-<br>21 | C 07 C 1/00<br>B 01 J |
| A | AT - B - 185 359 (RHEINPREUSSEN)<br>* Example *<br>-- | 1,2,18-<br>20 | |

The present search report has been drawn up for all claims

| Place of search | Date of completion of the search | Examiner |
|---|---|---|
| VIENNA | 26-05-1986 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

**European Patent Office**

# EUROPEAN SEARCH REPORT

Application number

–2–

| | DOCUMENTS CONSIDERED TO BE RELEVANT | | EP 86102802.5 |
|---|---|---|---|

| Category | Citation of document with indication, where appropriate, of relevant passages | Relevant to claim | CLASSIFICATION OF THE APPLICATION (Int. Cl.4) |
|---|---|---|---|
| A | AT – B – 175 878 (RHEINPREUSSEN) <br> * Examples 2,3 * <br> – – – – | 1,2,18-21 | |
| | | | **TECHNICAL FIELDS SEARCHED (Int. Cl.4)** |

The present search report has been drawn up for all claims

| Place of search | Date of completion of the search | Examiner |
|---|---|---|
| VIENNA | 26-05-1986 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

  & : member of the same patent family, corresponding document

EPO Form 1503 03.82